# EUROPÄISCHE PATENTSCHRIFT

(11) **EP 1 342 483 B1**
(45) Veröffentlichungstag und Bekanntmachung des Hinweises auf die Patenterteilung: **04.05.2005**
(21) Anmeldenummer: 03012949.8
(22) Anmeldetag: 28.05.1994
(51) Int. Cl.: A61M 15/00, B65D 83/04

(54) **Inhalator**
Inhalator
Inhalateur

(30) Priorität: 03.06.1993 DE 4318455
(43) Veröffentlichungstag der Anmeldung: 10.09.2003
(62) Teilanmeldung aus: 98123206.9
(73) Patentinhaber: BOEHRINGER INGELHEIM PHARMA GMBH & CO. KG, 55216 Ingelheim am Rhein (DE)
(72) Erfinder: Hochrainer, Dieter, 57392 Oberkirchen (DE); Kinneir, Ross, Bristol BS8 2UN (GB)

(56) Entgegenhaltungen:
- EP-A- 0 028 162
- EP-A- 0 129 985
- EP-A- 0 333 334
- WO-A-82/01470
- WO-A-91/02558

## Beschreibung

Die Erfindung betrifft ein Inhalationsgerät, in dem mit mikronisiertem Arzneimittel gefüllte Kapseln benutzt werden.

Inhalationsgeräte für die Pulverinhalation, mit deren Hilfe Arzneimittelpulver aus Kapseln inhaliert werden können, sind weithin in Gebrauch. Einige Versionen sind so ausgelegt, daß sie nur eine Kapsel aufnehmen konnten (DE-A 14 91 715; DE-A 33 45 722).

Um dem Patienten das Nachfüllen zu erleichtern, gibt es Inhalatoren, in denen mehrere Kapseln bereitgehalten werden können (z.B. DE-A 39 27 170). Die zur Aufnahme der Kapseln bestimmten Kammern oder Vertiefungen haben einen größeren Durchmesser als die Kapseln.

EP 0 028 162 offenbart einen Inhalator mit einem Förderband für mehrere Kapseln als integraler Bestandteil einer automatischen Öffnungsvorrichtung für die Kapseln. Das Förderband enthält Öffnungen oder Vertiefungen, die die Kapseln fest einklemmen, so daß ein zerstörungsfreies Herausnehmen der Kapseln nicht möglich ist. Zum Aufbewahren von Ersatzkapseln für ein manuelles Aufladen offenbart EP 0 028 162 auch einen becherförmigen Sammelbehälter ohne spezielle Haltevorrichtungen.

Beim Hantieren mit dem geöffneten Inhalator können die Kapseln leicht herausfallen, weil sie in den einzelnen Kammern nicht fixiert sind. Besonders beim Asthmaanfall, bei dem der Patient rasch die Inhalation vornehmen will, ist es störend, wenn die Kapseln nur lose im Inhalator liegen, so daß der Patient auch darauf achten muß, daß die Kapseln während der Benutzung nicht aus dem Gerät fallen. Andererseits ist es erwünscht, entleerte Kapseln sofort nach Gebrauch zu entfernen, ohne daß gleichzeitig die noch gefüllten Kapseln herausfallen, damit jeweils ohne weiteres festzustellen ist, wie groß der Vorrat an gefüllten Kapseln im Gerät noch ist.

Die Erfindung wird durch Anspruch 1 definiert.

Bevorzugt ist eine Kapselhalterung, die einerseits die Kapseln so fixiert, daß sie beim üblichen Hantieren damit nicht herausfallen, andererseits aber eine leichte Entnahme ermöglicht, und zwar unabhängig davon, mit welchem Ende die Kapseln in die Vertiefungen gesteckt werden.

Bekanntlich bestehen die zu therapeutischen Zwecken verwendeten Arzneimittelkapseln aus zwei Teilen; jeder der Teile hat zylindrische Form und hat an einem Ende einen halbkugelförmigen Abschluß. Der Innendurchmesser der beiden Zylinder ist so gewählt, daß beim Zusammenstecken mit den offenen Seiten eine relativ feste Verbindung erreicht wird. Der Außendurchmesser der beiden zylindrischen Teile ist verschieden. Bei einer handelsüblichen Kapsel der Größe 3 beispielsweise beträgt der Durchmesser des Oberteils 5,83, der Durchmesser des Unterteils 5,57 mm. Die Kapseln bestehen aus elastischem Material, vorzugsweise aus Hartgelatine.

Zylindrische Vertiefungen, die den äußeren Abmessungen der Kapseln entsprechen, lösen die erfindungsgemäße Aufgabe nicht, weil sie jeweils für eine Kapselhälfte zu eng oder zu weit sind.

Es wurde nun eine Kapselhalterung für Arzneimittelkapseln entwickelt, in welche die Kapseln sowohl mit ihrem Ober- als auch mit ihrem Unterteil gleich tief eingesteckt werden können, wobei sie einerseits ausreichend fest sitzen, um nicht herauszufallen, andererseits leicht genug entnehmbar sind, damit die Teile der Kapseln bei der Entnahme nicht versehentlich auseinandergezogen werden.

Die bevorzugte Halterung besteht aus einer Vertiefung, in welche die Kapsel in Richtung ihrer Längsachse hineingesteckt wird, wobei die Wandung der Vertiefung mindestens drei parallel zur Längsachse in ungleichem Abstand voneinander angeordnete Rippen aufweist, zwischen denen die Kapsel unter Verformung eingeklemmt wird.

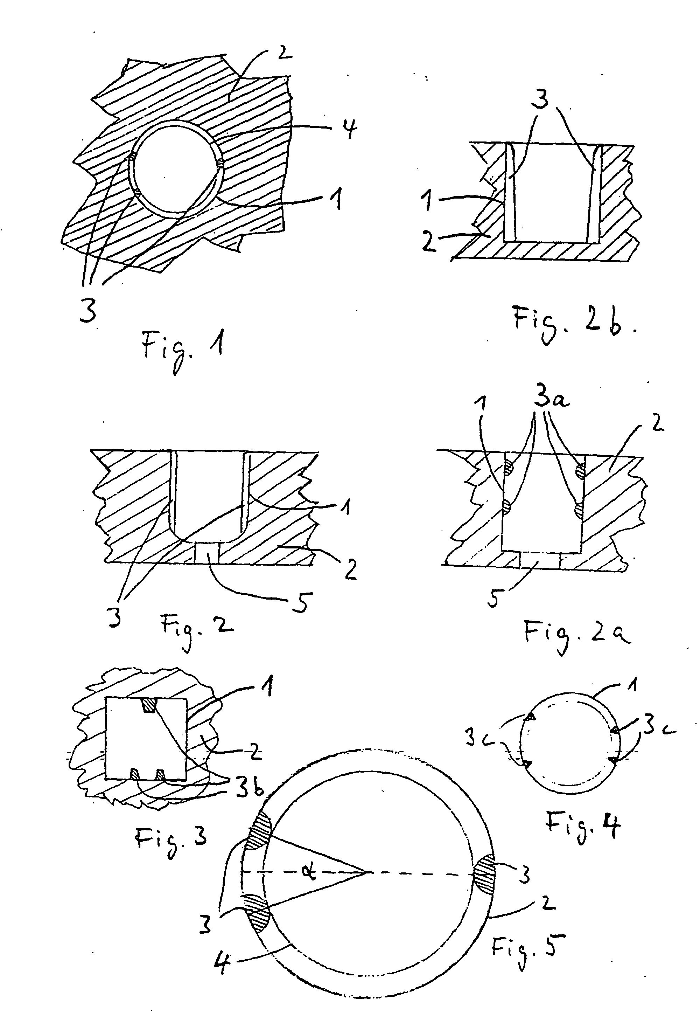

Eine Kapselhalterung dieser Art ist in den Figuren 1 bis 6 dargestellt.
Figur 1 zeigt eine bevorzugte Kapselhalterung im Querschnitt.
   Die Vertiefung 1 mit kreisförmigem Querschnitt in der Platte 2 weist drei Rippen 3 auf, von denen zwei verhältnismäßig nahe beeinander liegen und die dritte gegenüber. Zwischen den Rippen läßt sich ein Kreis 4 einbeschreiben, dessen Durchmesser geringfügig kleiner ist als der Außendurchmesser des unteren (dünneren) Kapselteils. Beim Einschieben zwischen die Rippen wird die Kapsel leicht verformt. Zweckmäßigerweise werden die Rippen an ihrem äußeren Ende abgerundet oder abgeschrägt, was das Einführen der Kapseln erleichtert und eine Beschädigung der Kapseln beim Einführen verhindert.
   Der Querschnitt der Rippen läßt viele Abwandlungen zu, meist ist er halbkreisförmig bzw. abgerundet, er kann aber auch z.B. dreieckig oder viereckig sein, wobei die Höhe der Rippen vorzugsweise gleich ist, aber nicht gleich sein muß. Entsprechendes gilt für die Breite der Rippen. Die Fläche, mit der sich Kapseln und Rippen berühren, sollte relativ klein sein, damit auch nach längerem Aufbewahren in der Halterung die Kapsel noch gut entnommen werden kann und nicht verklebt. Die Vertiefung kann sich (bei konstanter Rippenhöhe) etwas verengen, das heißt leicht konisch geformt sein; auch können die Rippen zum unteren Ende der Vertiefung hin etwas höher werden, so daß auch in diesem Fall (bei konstantem Querschnitt der Vertiefung) eine Verringerung des Zwischenraums zwischen den Rippen erreicht wird.
Figur 2 zeigt einen senkrechten Schnitt entlang der Längsachse einer Kapselhalterung, wobei in der Vertiefung 1 der Platte 2 die Rippen 3 zu erkennen sind. Die Vertiefung 1 besitzt im allgemeinen unten eine Öffnung 5, welche die Reinigung erleichtert.
Wie Figur 2a zeigt, können die Rippen auch durch entsprechend angeordnete Noppen 3a ersetzt werden.
In Figur 2b wächst die Höhe des Rippenquerschnitts nach unten, so daß sich nach unten hin ein geringerer Abstand der Rippen voneinander ergibt.
Figur 3 zeigt ein Beispiel für eine Kapselhalterung mit rechteckigem Querschnitt und einer andersartigen Gestaltung der Rippen (3b).

Eine größere Zahl von Rippen läßt Figur 4 erkennen, wobei die Rippen 3c einen dreieckigen Querschnitt haben.

Um (unter sonst gleichen Voraussetzungen) eine ausreichende Verformung zu ermöglichen, sind die Rippen - bezogen auf den einbeschriebenen Kreis - nicht in gleichen Abständen angeordnet. Dies soll in Figur 5 veranschaulicht werden. Der Winkel α zwischen den beiden enger benachbarten Rippen 3 beträgt etwa 30 bis 60°, vorzugsweise 35 bis 50°. Besonders bewährt sich ein Winkel von ca. 40°. Die gegenüberliegende Rippe liegt auf dem Durchmesser, der den Winkel α halbiert oder ist allenfalls geringfügig seitlich versetzt. Bei einem Winkel von z. B. 40° zentriert sich die Kapsel beim Einführen von selbst, während sie bei zu kleinem Winkel schräg stehen kann, so daß u. U. das herausnehmen behindert sein kann, insbesondere wenn mehrere Halterungen dicht nebeneinander vorgesehen sind.

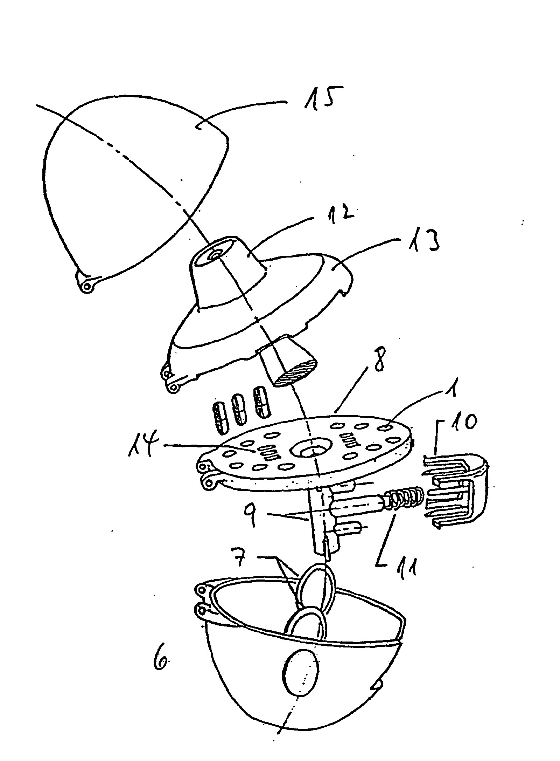

Aus Figur 6 ist zu entnehmen, wie ein Inhalator aufgebaut sein kann, in den bevorzugte Kapselhalter integriert sind.

In einem Unterteil 6 mit zwei Fenstern 7 befindet sich die Platte 8, die mit der Inhalationskammer 9 verbunden ist und in der sich auch 12 Kapselhalterungen 1 befinden. Zum Öffnen der Kapseln in der Kapselkammer 9 dient der mit zwei speziell geschliffenen Nadeln versehene Knopf 10, der gegen den Druck der Feder 11 eingedrückt wird und dabei die Kapsel in der Kammer an zwei Stellen aufschneidet bzw. aufsticht. Beim Inhalieren durch das Gerät mittels des Mundrohrs 12, das mit dem Oberteil 13 verbunden ist, gelangt die Luft durch die Öffnungen 14 in das Unterteil 6 und von dort am unteren Ende in die Kapselkammer 9, die so gebaut ist wie die Kapselkammer in dem Inhalator gemäß DE-A 3927170. Das Gerät wird durch einen Deckel verschlossen, der klappbar mit dem Unterteil 6, der Platte 8 und dem Oberteil 13 verbunden ist, so daß bei geschlossenem Deckel Staub nicht in das Gerät eindringen kann.

## Patentansprüche

1. Inhalator für die Inhalation Arzneimittelpulver aus Kapseln **gekennzeichnet durch**
- ein nach oben hin offenes, becherförmiges Unterteil (6), welches in der Ummantelung zwei gegenüber liegende Fenster (7) aufweist und am Rand der Öffnung ein erstes Scharnierelement hat,
- eine Platte (8), welches die Öffnung des Unterteils bedeckt und ein zweites Scharnierelement aufweist,
- eine Kapselkammer (9), die senkrecht zur Plattenebene an der zum Unterteil (6) weisenden Seite der Platte (8) ausgebildet ist und an der ein gegen eine Feder (11) beweglicher Knopf (10) vorgesehen ist, wobei der Knopf mit zwei geschliffenen Nadeln versehen ist,
- ein Oberteil (13) mit einem Mundrohr (12) und einem dritten Scharnierelement, sowie
- einen Deckel (15), der ein viertes Scharnierelement aufweist,
- wobei die Scharnierelemente eins des Unterteils (6), zwei der Platte (8), drei des Oberteils (13) und vier des Deckels (15) miteinander verbunden sind.

2. Inhalator nach Anspruch 1, wobei die Platte (8) Kapselhalter in Form von Vertiefungen (1) aufweist, deren Wandungen parallel zur Längsachse einen größeren Abstand voneinander haben als der größten Kapseldurchmesser, und die Wandungen in ungleichem Abstand mindestens drei parallel zur Längsachse verlaufende Rippen (3; 3a; 3b; 3c) aufweisen, zwischen denen eine Kapsel eingeklemmt werden kann und die zur Längsachse orientierten Flächen oder Kanten der Rippen einen einbeschriebenen Zylinder (4) definieren, der einen geringfügig kleineren Durchmesser hat als das Kapselteil mit dem kleinsten Kapseldurchmesser.

3. Inhalator nach Anspruch 1 und 2, wobei die Platte (8) Lufteinlaßöffnungen (14) aufweist.

## Claims

1. Inhaler for inhaling powdered pharmaceutical preparations from capsules, **characterised by**
- a cup-shaped lower part (6) which is upwardly open, which has two opposite ports (7) in the casing and has a first hinge element at the edge of the opening,
- a plate (8) which covers the opening in the lower part and comprises a second hinge element,
- an capsule chamber (9) which is formed perpendicularly to the plane of the plate on the side of the plate (8) facing the lower part (6) and on which is provided a button (10) which is movable counter to a spring (11), the button being provided with two sharpened pins,
- an upper part (13) with a mouth tube (12) and a third hinge element, and
- a lid (15) which comprises a fourth hinge element,
- the hinge elements of (one) the lower part (6) (two) the plate (8), (three) the upper part (13) and (four) the lid (15) being joined to one another.

2. Inhaler according to claim 1, wherein the plate (8) comprises capsule holders in the form of recesses (1) the walls of which, parallel to the central axis, are at a spacing from one another which is greater than the maximum capsule diameter, and the walls having at least three ribs (3; 3a; 3b; 3c) at unequal spacings from one another, running parallel to the longitudinal axis, between which a capsule can be gripped, and the surfaces or edges of said ribs, oriented towards the longitudinal axis, defining an inscribed cylinder (4) which is slightly smaller in diameter than the part of the capsule having the smallest diameter.

3. Inhaler according to claims 1 and 2, wherein the plate (8) has air inlet openings (14).

## Revendications

1. Inhalateur pour l'inhalation de médicaments sous forme de poudre en capsules,
**caractérisé par**
- une partie inférieure (6) en forme de bac, ouverte vers le haut, laquelle comprend deux fenêtres (7) opposées l'une à l'autre dans le revêtement et présente un premier élément articulé sur le bord de l'ouverture,
- une plaque (8), laquelle recouvre l'ouverture de la partie inférieure et comprend un deuxième élément articulé,
- une chambre pour le capsule (9), qui est conçue perpendiculairement au plan de la plaque sur le côté de la plaque (8) orienté vers la partie inférieure (6) et qui est prévue sur la un bouton (10) mobile à l'encontre d'un ressort (11), le bouton étant dotée de deux aiguilles affilées,
- une partie supérieure (13) comprenant un tuyau de sortie (12) et un troisième élément articulé, ainsi que par
- un couvercle (15), qui comprend un quatrième élément articulé,
- les éléments articulés premièrement de la partie inférieure (6), deuxièmement de la plaque (8), troisièmement de la partie supérieure (13) et quatrièmement du couvercle (15) étant reliés les uns aux autres.

2. Inhalateur selon la revendication 1, dans lequel la plaque (8) comprend des porte-capsules sous forme de renfoncements (1), dont les parois sont espacées parallèlement à l'axe longitudinal d'une distance supérieure au plus grand diamètre de capsule, et les parois comprennent à une distance inégale au moins trois nervures (3 ; 3a; 3b ; 3c) s'étendant parallèlement à l'axe longitudinal, entre lesquelles peut être coincée une capsule, et les surfaces ou arêtes des nervures orientées vers l'axe longitudinal définissent un cylindre inscrit (4) qui présente un diamètre légèrement plus petit que la partie de capsule présentant le diamètre de capsule le plus petit.

3. Inhalateur selon la revendication 1 et 2, dans lequel la plaque (8) comprend des ouvertures d' admission d'air (14).
